# EUROPEAN PATENT APPLICATION

(11) **EP 4 302 811 A1**
(43) Date of publication of application: **10.01.2024**
(21) Application number: 22183939.2
(22) Date of filing: 08.07.2022
(51) Int. Cl.: A61M 25/00, A61M 25/06, A61M 25/04

(54) **URINARY CATHETER ASSEMBLY AND INTRODUCER DEVICE FOR USE IN SUCH AN ASSEMBLY**

(71) Applicant: Wellspect AB, 431 21 Mölndal (SE)
(72) Inventor: UTAS, Jan, 434 95 Kungsbacka (SE); LOVMAR, Martin, 431 69 Mölndal (SE); TÖRNEROOS, Hans, 22120 Mariehamn (FI); GALLOWAY, Niall, 431 21 Mölndal (SE)
(74) Representative: Lind Edlund Kenamets Intellectual Property AB

(57) **Abstract**

A urinary catheter assembly comprising a urinary catheter (2) including a catheter shaft (21) and an introducer device (3). The introducer device (3) comprises a splitable tubular sheath (31), wherein the introducer device (3) is arranged to accommodate at least a part of the catheter shaft (21) for insertion into the urethra, and subsequent removal. At least a part of an outer surface of the tubular sheath (3) is provided with a hydrophilic coating/surface.

## Description

### TECHNICAL FIELD OF THE INVENTION

The present invention relates to a urinary catheter assembly and an introducer device for use in such a urinary catheter assembly.

### BACKGROUND OF THE INVENTION

The present invention relates to a urinary catheter assembly. Urinary catheters are commonly used for draining urine from the bladder. Urinary catheters can be of an indwelling type, for long term use, such as days or even weeks, or for intermittent use, whereby the catheters are used for a single draining procedure, typically lasting a few minutes.

Intermittent urinary catheters are e.g. used by a large group of persons for self-catheterization, which is a daily-life procedure, taking place several times a day. Many catheters, such as those for intermittent catheterization, are provided with a hydrophilic coating or the like, providing a smooth and slippery surface when wetted, for safe and comfortable insertion in the urinary canal.

A typical intermittent catheter also differs from an indwelling catheter primarily in that the intermittent catheter does not have a retention balloon or an associated inflation lumen. Rather, the intermittent catheter is typically a single-lumen device, with multiple drainage eyes at the proximal end and a funnel at the distal end.

A common type of indwelling catheters is Foley catheters. Foley catheterization is typically indicated for surgical and medical patients who require, at least temporarily, assisted bladder voiding. Common indications to catheterize a patient include acute or chronic urinary retention (which can damage the kidneys), medical procedures (i.e., surgeries) that may at least temporarily limit a patient's movement, the need for accurate monitoring of input and output (such as in an ICU), benign prostatic hyperplasia, incontinence, and the effects of various surgical interventions involving the bladder and prostate.

A standard Foley catheter design includes a balloon disposed at the proximal end of the catheter to anchor the catheter in the bladder. The catheter includes at least one lumen to drain urine from the bladder and at least one lumen to inflate the balloon, e.g. with air or sterile water. The proximal end of the Foley catheter includes at least two ports in communication with the two lumens. A first port is connected to the drainage lumen and has an interface with fittings for drainage and sampling. A second port is connected to the inflation lumen with a valve to ensure the inflation fluid remains within the lumen and balloon once filled. The tip of a standard Foley catheter extends beyond the sides of the balloon into the bladder and includes one or more apertures or "eyes" to drain fluids and debris from the bladder.

It is also known to use other forms of retention elements than inflatable balloons, such as retention wings, as discussed e.g. in US 7264609 and WO 21/224248, and a spiral shaped insertion end, as discussed e.g. in WO 96/02214.

However, there are some problems associated with indwelling urinary catheters. The catheters are relatively complex to produce. In Foley catheters, manufacturing of the balloon is relatively complex, and the catheter shaft needs to have at least two lumens - one for drainage of urine and one for inflation/deflation of the balloon. Indwelling catheters having other types of retention elements are also often complicated and costly to produce, and are also often complicated to use, since the retention elements need to be manipulated to allow insertion and extraction, and at the same time provide retention to maintain the catheter in place when inserted into the bladder.

Further, intermittent catheters can be inserted and extracted easily and without any pain for the user, due to the hydrophilic coating commonly used, providing the catheter surface with a very low friction. However, such hydrophilic coatings can normally not be used for indwelling catheters, since the hydrophilic coatings will over time dry out in the urethra, and thereby stick to the urethral wall. Hereby, the friction will after some time increase dramatically, making removal of the catheter very difficult and painful when this has occurred. To this end, indwelling catheters are instead often provided with a lubricating gel or the like to decrease friction, but even with such lubrication, the friction will still not be as low for hydrophilic intermittent catheters.

A further problem concerning indwelling catheters is that relatively stiff and rigid catheter shafts, which are often used for intermittent catheters, may feel uncomfortable and even painful when used for an extensive period of time, such as hours, days or even longer, due to the pressure exerted by the catheter on bends and curvature along the urethra. To this end, it would generally be desirable to use softer, more flexible catheter shafts for indwelling catheters. However, such softer, more flexible catheter shafts are more difficult to insert, and there is also a risk that the catheter shaft may kink during insertion.

Thus, there is still a need for improved urinary catheter assemblies, and in particular indwelling urinary catheter assemblies. The assembly should preferably be relatively simple and cost-efficient to produce. Further, the assembly should be easy and quick to prepare for use, and allow intuitive handling for the user, and at the same time be simple to insert and use in a painless way. In particular, there is a need for improved indwelling catheters and indwelling urinary catheter assemblies addressing all, or at least some, of the above-discussed problems and drawbacks.

### SUMMARY OF THE INVENTION

It is an object of the present invention to provide a urinary catheter assembly addressing the above-discussed needs and to alleviate at least some of the above-discussed drawbacks.

This object is obtained by a urinary catheter assembly as defined in the enclosed claims.

According to a first aspect of the invention, there is provided a urinary catheter assembly comprising a urinary catheter including a catheter shaft, and an introducer device including a splitable tubular sheath, wherein the introducer device is arranged to accommodate at least a part of the catheter shaft, and wherein at least a part of an outer surface of the tubular sheath is provided with a hydrophilic coating/surface.

With this urinary catheter assembly, the catheter shaft can be inserted into the urethra of the user together with the introducer device, while maintaining the catheter shaft in the tubular sheath of the introducer device. This stabilizes the catheter shaft during insertion, and makes it more rigid, thereby facilitating insertion and avoiding the risks of kinking and the like.

Once the catheter has been inserted, the splitable tubular sheath can be split and the introducer device be removed from the urethra, thereby releasing the catheter and leaving it in place in the urethra.

Thus, the combination of the catheter shaft and the tubular sheath has increased stability and is easier to push into the urethra than what had been the case with only the catheter shaft, without the introducer device. This also makes it possible to use a softer, more flexible catheter shafts, which makes the catheters more comfortable during use, in particular when used over an extended period of time, and which reduces the risk of pain. For example, it is hereby possible to use catheter shafts being so soft and flexible that they could not be inserted into the urethra on their own, without the assistance and stabilization from the introducer device. To this end, the catheter shaft can be made of softer materials than used conventionally, or with a thinner wall thickness.

The catheter shaft can e.g. be made of silicone rubber, latex rubber, polyurethane (PUR) or thermoplastic elastomers (TPE). However, other materials are also feasible. Further, more than one material may be used, and arranged e.g. mixed to a blend or arranged in separate parts, such as in separate layers.

Further, since the introducer device is used only during insertion of the catheter, and thereafter removed, it is present in the urethra only during a very limited time period. Consequently, it is possible to use a hydrophilic coating/surface on the tubular sheath of the same type as is presently used on intermittent catheters, thereby greatly facilitating the insertion process, and reducing the risk of causing pain and damage to the urethra. Since the catheter shaft is at least partly covered by the tubular sheath during insertion, the catheter shaft need not be coated, or otherwise treated to reduce friction.

Thus, the present invention uniquely provides a urinary catheter assembly allowing simple and easy insertion of the catheter, and at the same time a catheter which is gentle and comfortable to use for a prolonged period of time, such as days, weeks or even months.

The urinary catheter assembly is in particular suitable for use with indwelling catheters, also referable to as retention catheters. However, it may also be used together with intermittent catheters, for short time use.

The introducer device is preferably arranged to accommodate at least an insertable part of the catheter shaft. Hereby, the tubular sheath covers at least the whole part of the catheter that is going to be inserted during ordinary use. Further, the hydrophilic coating/surface is preferably provided over at least the part of the tubular sheath that covers the insertable part of the catheter shaft, i.e. at least the insertable part of the introducer device. Hereby, introduction is further facilitated.

The tubular sheath preferably has an inner diameter greater than an outer diameter of the catheter shaft, and preferably only slightly greater, and more preferably essentially corresponding to the outer diameter of the catheter shaft. Hereby, the introducer device closely surrounds the catheter shaft, minimizing the outer diameter of the catheter and introducer device arrangement. Minimizing the outer diameter of the insertable parts further facilitates insertion.

In an embodiment, the introducer device comprises a first end and a second end remote from the first end, and at least one tear line extending between the first and second ends. The tear line may e.g. be a line forming a weakening in the material, such as a line of perforations, a line made of a different, weaker material, a line along which the wall thickness is thinner, e.g. by being partly cut through, etc. In an embodiment, the cut through or perforations extend over more than 50% of the wall thickness of the tubular sheath, and preferably more than 70%, such as 80-90%. When a force is applied on the introducer device, the tear line will preferably be the part of the introducer device that is separated, thereby forming the split. The splitting enables the removal of the introducer device from the catheter when it has been inserted into the urethra.

In one embodiment only one tear line is provided. However, more than one tear lines may also be used. In one embodiment two tear lines are provided. The two tear lines may extend between the first and second ends, the tear lines being separated from each other in a circumferential direction. For example, the two tear lines may be arranged on opposite sides of the tubular sheath. By provision of two tear lines, the tubular sheath may be split into two separated parts. More than two tear lines may also be provided.

The tear line(s) may extend generally along the length direction of the tubular sheath. However, other paths are also possible, such as helical paths and the like.

In a preferred embodiment, the tear line(s) extend over the entire length of the tubular sheath, and/or over the entire length of the introducer device.

The tubular sheath is preferably made of a flexible material. The flexibility is preferably such that the sheath can be compressed onto a catheter shaft by moderate to low finger pressure, e.g. using the thumb and the index finger or middle finger.

The introducer device may further comprise at least one gripping element arranged at, or in the vicinity of, a non-insertable end of the introducer device. The gripping device may e.g. be in the form of handles or flanges protruding out from the tubular sheath. In an embodiment, the gripping device may protrude outwardly, at least partially in a radial, lateral direction. In an embodiment, two gripping devices are provided, preferably arranged separated or distributed along the circumference of the tubular sheath, and preferably at opposite sides, and protruding in opposite directions. However, only one gripping device may also be used, or more than two gripping devices. In one embodiment, the number of gripping devices corresponds to the number of tear lines provided in the tubular sheath. In such an embodiment, the gripping devices are preferably arranged between the tear lines.

Since the gripping element(s) is/are arranged on the introducer device, they will be removed together with the introducer device, and will consequently not cause any inconvenience during a prolonged use of the catheter. Consequently, the introducer device, and in particular the gripping elements can be relatively large, to provide better gripping functionality.

The gripping elements may be in the form of a gripping tab, a gripping ring, etc. The gripping elements may be provided with a texture, such as corrugations, to increase friction when gripped.

The tubular sheath may have a Shore A hardness which is greater than the Shore A hardness of the catheter shaft of the urinary catheter. In an embodiment, the tubular sheath has a Shore A hardness exceeding 65, and preferably in the range 70-90, and the catheter shaft has a Shore A hardness of 60 or lower, and preferably in the range 40-60.

The urinary catheter may further comprise an expandable retention element. The retention element may be an inflatable element, such as an inflatable balloon, but may also be a non-inflatable retention element. The introducer device is preferably arranged to maintain the expandable retention element in a compressed state when accommodating the catheter shaft. Hereby, the retention element is compressed by the introducer device, for easy and simple insertion into the urethra. When the introducer device is subsequently removed, the retention element is allowed to expand, and will then serve to maintain the catheter in place.

The catheter shaft, when accommodated in the introducer device, may have an insertable tip portion protruding out from the tubular sheath of the introducer device. The protruding tip may have a maximum outer diameter which is greater than the outer diameter of the tubular sheath. The protruding tip may e.g. be in the shape of a ball. The insertable tip of the catheter may e.g. be a rounded tip, providing a gentle and non-harmful insertion into the urethra.

However, alternatively, the tubular sheath may extend all the way to the insertable tip of the catheter, and may also extend beyond the insertable tip.

In another embodiment, the tip of the introducer device may be made of a soluble material that will disintegrate upon contact with urine.

The hydrophilic coating/surface preferably comprises polyvinylpyrrolidone (PVP).

The hydrophilic coating/surface may be arranged as a hydrophilic coating arranged on a substrate of the tubular sheath. However, the hydrophilic coating/surface may alternatively be arranged as an integrated part of the tubular sheath, such as an integrated layer, or alternatively, the entire tubular sheath may be made of a hydrophilic material. The hydrophilic coating/surface is preferably arranged to provide low friction when wetted.

In an embodiment the hydrophilic coating/surface may be a surface provided with a hydrophilic coating, for example made in accordance with EP 0 093 093 and EP 0 217 771, said documents hereby being incorporated by reference in their entirety.

Even though PVP is the preferred hydrophilic material, other hydrophilic materials may be used, such as hydrophilic polymers selected from polyvinyl compounds, polysaccharides, polyurethanes, polyacrylates or copolymers of vinyl compounds and acrylates or anhydrides, especially polyethyleneoxide, polyvinyl-pyrrolidone, heparin, dextran, xanthan gum, polyvinyl alcohol, hydroxy propyl cellulose, hydroxy propyl methyl cellulose, methyl cellulose, copolymer of vinylpyrrolidone and hydroxy ethylmethyl acrylate or copolymer of polymethylvinyl ether and maleinic acid anyhydride. Further, hydrophilic coating/surface is to be understood in a broad sense, and in embodiments the hydrophilic coating/surface may comprise an entire tubular sheath formed of a hydrophilic material.

The tubular sheath may further be provided with a coating also on an inner surface, the coating preferably being a hydrophilic coating. Such an inner coating will facilitate the removal of the introducer device, since it will lower the friction between the catheter and the tubular sheath. However, alternative measures to lower the friction are also feasible, such as providing a lubricating gel or other friction reducing fluids between the introducer device and the catheter shaft.

The urinary catheter assembly may further comprise a package accommodating the urinary catheter and the introducer device. The introducer device is preferably pre-arranged on the urinary catheter, thereby forming a ready-to-use kit.

The assembly may further comprise a wetting fluid arranged in the package. In one embodiment, the wetting fluid is arranged in a separate compartment of the package, to be released into contact with the hydrophilic coating/surface prior to use. In another embodiment, the wetting fluid is maintained in constant contact with the hydrophilic coating/surface, thereby maintaining the coating/surface in an activated, ready-to-use state.

The package may be of a flexible material, such as foil material. However, the package may also be of a more rigid material, such as rigid plastic material.

The package may, in a closed position, provide a sterile and moisture proof compartment for the introducer device and the urinary catheter.

The catheter may have a connector, funnel shaped end or flared end arranged at the non-insertable part, such as a flared connector. The catheter preferably has an internal lumen, extending from one or more drainage openings, so-called eyes or eyelets, arranged at or in the vicinity of the proximal insertion end, to a discharge outlet arranged at or in the vicinity of the distal end. A drainage opening may be arranged at the proximal tip of the elongate shaft, i.e. forming a centrally arranged opening in the longitudinal extension of the internal lumen. Additionally, or alternatively, one or more drainage openings may be formed in the sidewall of the elongate shaft, to debouch in a direction radially outwards. In one embodiment, the elongate shaft has a rounded, closed proximal end, and one or more drainage openings arranged in the sidewall of the elongate shaft.

Further, the distal part may, at least on a part thereof, have larger cross-sectional dimensions than the catheter shaft. The distal part may e.g. be flared or funnel shaped, increasing in dimension towards the distal end, thereby enabling connection of a tube, collection bag or the like.

The assembly may further comprise a guide arrangement, such as a ring encircling the introducer device, at a position in the vicinity of the non-insertable part of the catheter. The guide arrangement is preferably arranged at a non-insertable part of the assembly. The guide arrangement may be arranged to maintain the introducer device intact until it has passed the guide arrangement, when being pulled to be removed and split. This ensures that the splitting of the introducer device occurs after the ring, at a position outside the urethra. This is of advantage, since splitting of the introducer device may otherwise tend to occur at the urethra orifice, or to some extent inside the urethra. If this happens, it may exert stress on the urethral wall, close to the orifice, which may feel uncomfortable. However, the guiding element may also be omitted.

The assembly may further comprise a cutting aid arranged in the vicinity of the non-insertable part of the catheter, aiding in severing the introducer device. The cutting aid may e.g. be arranged connected to or integrated with the optional guide arrangement. The cutting aid may e.g. comprise a pin, a blade or the like, penetrating into the introducer device, thereby assisting the splitting of the introducer device as it is pulled out passing the cutting aid.

According to another aspect of the invention, there is provided an introducer device for insertion of a urinary catheter comprising a splitable tubular sheath, wherein at least a part of an outer surface of the tubular sheath is provided with a hydrophilic coating/surface.

### BRIEF DESCRIPTION OF THE DRAWINGS

The present invention will be described in more detail with reference to the appended drawings, showing currently preferred embodiments of the invention.
Fig. 1 is a sideview of a urinary catheter useable in a urinary catheter assembly in accordance with an embodiment of the present invention.
Fig. 2 is a schematic sideview of an introducer device in accordance with an embodiment of the present invention.
Fig. 3 is a sideview of a urinary catheter assembly, including a catheter and an introducer device, in accordance with an embodiment of the present invention.
Fig. 4 is a schematic sideview of an introducer device in accordance with another embodiment of the present invention.
Figs. 5a-d are cross-sectional views of an introducer device in accordance with different embodiments.
Fig. 6 is a schematic illustration of a guide element useable with an introducer device in accordance with the invention.
Fig. 7 is a schematic illustration of an extruder arrangement for production of an introducer device, in accordance with an embodiment of the invention.
Figs. 8a-d are schematic illustrations of various retention elements useable on a urinary catheter in embodiments of the invention.
Figs. 9a-d are schematic illustrations of various tip configurations for the urinary catheter assembly of various embodiments.
Fig. 10 is a schematic illustration of a catheter kit assembly in accordance with an embodiment of the present invention.

### DETAILED DESCRIPTION OF CURRENTLY PREFERRED EMBODIMENTS

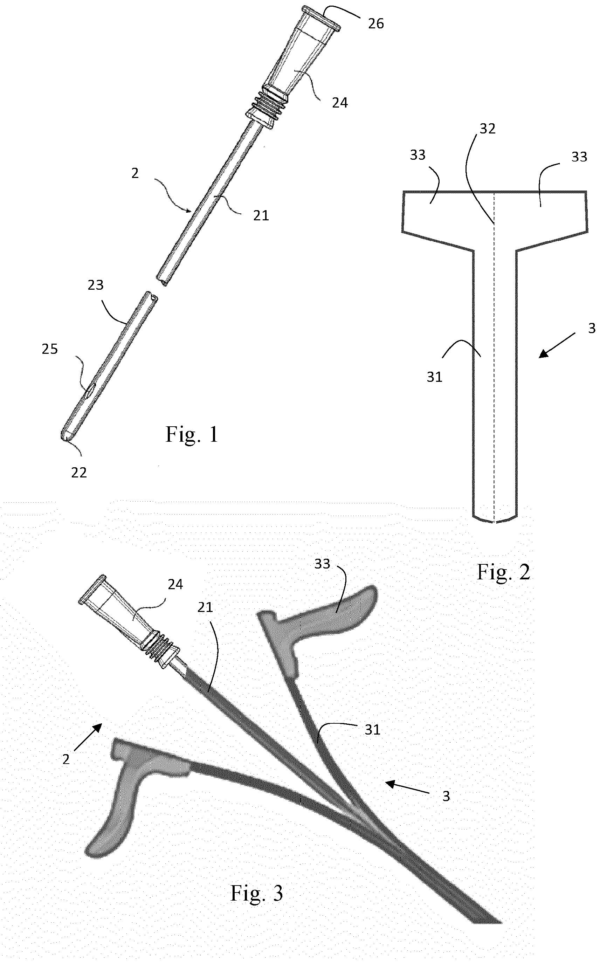

A urinary catheter 2 as illustrated in Fig. 1 comprises an insertable section 21, comprising an insertable, proximal part, with an insertion tip 22, on a catheter shaft 23, and a non-insertable section 24, a distal part, forming a connector part. The non-insertable section 24 preferably has a larger diameter than the insertable section 21 at least on a part thereof. The rear end of the non-insertable section may be flared or funnel-shaped, and may be arranged to be connected to a tapered connection part of a urine collection bag or the like. However, the non-insertable section may alternatively have a relatively uniform cross-sectional area.

At least a part of the insertable section 21 forms an elongate shaft with an insertable length to be inserted into a natural or artificial body opening of a user, such as into a urethra of the user.

The insertable section comprises an insertion tip, which may be a closed, rounded end. Further the insertable section may comprise one or more drainage openings 25, arranged in the vicinity of the insertion tip, so called catheter eyes or eyelets, leading into a lumen extending through the catheter, and into a discharge outlet 26 arranged at the rearward end of the non-insertable section 24.

The insertable section may be 80-140 mm for a female user and 200-350 mm for a male user.

In an embodiment, the urinary catheter assembly comprises a catheter as discussed in the foregoing and an introducer device 3, as shown in Fig. 2. The introducer device includes a splitable tubular sheath 31, arranged to accommodate at least a part of the catheter shaft 23. The tubular sheath is preferably arranged to accommodate the whole catheter shaft, or at least the insertable part thereof, or at least s substantial part of the catheter shaft and/or the insertable part.

At least a part of an outer surface of the tubular sheath is provided with a hydrophilic coating/surface, and preferably at least the outer surface of the insertable part of the introducer device. In a preferred embodiment, essentially the entire outer surface of the tubular sheath may be provided with the hydrophilic coating/surface. The hydrophilic coating/surface preferably comprises polyvinylpyrrolidone (PVP). However, other hydrophilic polymers are also feasible, and could also be used for the coating/surface, to provide low friction, as is per se known in the art. The hydrophilic coating/surface may be arranged as a hydrophilic coating arranged on a substrate of the tubular sheath. However, the hydrophilic coating/surface may alternatively be arranged as an integrated part of the tubular sheath, such as an integrated layer, or alternatively, the entire tubular sheath may be made of a hydrophilic material. The hydrophilic coating/surface is preferably arranged to provide low friction when wetted.

The tubular sheath 31 preferably has an inner diameter essentially corresponding to an outer diameter of the catheter shaft. Hereby, the introducer device closely surrounds the catheter shaft, minimizing the outer diameter of the catheter and introducer device arrangement.

In an embodiment, the introducer device comprises a first end and a second end remote from the first end, and at least one tear line 32 extending between the first and second ends. The tear line(s) preferably extends along a substantial part of the length of the tubular sheath, and preferably over the entire length of the tubular sheath, and most preferably along the entire length of the introducer device.

The tear line 32 may e.g. be a line forming a weakening in the material, such as a line of perforations, a line made of a different, weaker material, a line made of an interface between two different materials, a line along which the wall thickness is thinner, e.g. by being partly cut through, etc. In an embodiment, the cut through or perforations extend over more than 50% of the wall thickness of the tubular sheath, and preferably more than 70%, such as 80-90%. When a force is applied on the introducer device, the tear line will preferably be the part of the introducer device that is separated, thereby forming the split. The splitting enables the removal of the introducer device from the catheter when it has been inserted into the urethra.

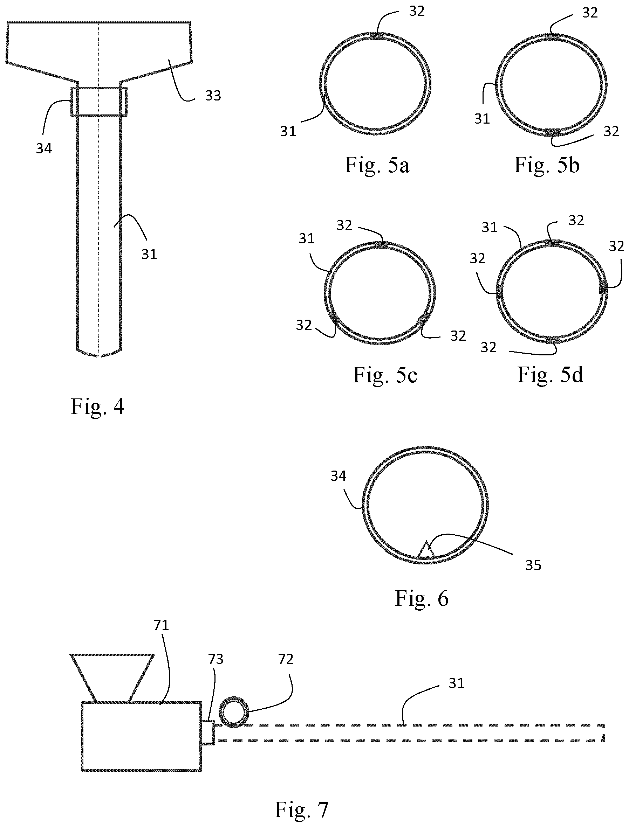

The introducer device may e.g. be produced by extrusion. In such a case, as shown in Fig. 7, the tear line may e.g. be formed by a cutting device 72, such as a roller with a cutting edge, arranged close to the extruder 71, and preferably adjacent or in the vicinity of the outlet nozzle 72 of the extruder 71. However, a fixed, non-rotatable cutting device may also be used, such as a sharp blade, a cutting tooth, or the like. Hereby, the cutting to form the tear line can be obtained while the extruded material is still warm and soft, which facilitates formation of the tear line, and also provides improved quality of the tear line surfaces and edges.

Alternatively, the introducer device can be produced in other ways, such by injection molding, etc. It is also possible to produce the introducer device as separate parts which are then connected together. For example, the tubular sheath may be formed by extrusion, and the gripping elements may be formed separately, e.g. by injection molding, and then connected to the tubular sheath.

However, alternatively, the cutting or perforation can be made any time after the extrusion, or the forming of the tubular sheath in other ways.

In case a coating is provided on the tubular sheath, the coating is preferably made after formation of the tear lines. However, it is also feasible to form the tear lines after the coating step.

In one embodiment only one tear line 32 is provided, as in the illustrative example of Fig. 5a. However, more than one tear lines may also be used.

In one embodiment, illustrated in Fig. 5b, two tear lines 32 are provided. The two tear lines 32 may extend between the first and second ends, the tear lines being separated from each other in a circumferential direction. For example, the two tear lines may be arranged on opposite sides of the tubular sheath, as in the illustrative example. However, other arrangements are also feasible. By provision of two tear lines, the tubular sheath may be split into two separated parts.

More than two tear lines 32 may also be provided. For example, as illustrated in Fig. 5c, three tear lines 32 may be provided, and preferably arranged equidistantly separated around the circumference of the tubular sheath. Alternatively, four tear lines 32 may be provided, as illustrated in Fig. d, and preferably arranged equidistantly from each other, in four cardinal directions, such as in a north-south-east-west arrangement.

The tear line(s) may extend generally along the length direction of the tubular sheath. However, other paths are also possible, such as helical paths and the like.

In a preferred embodiment, the tear line(s) extend over the entire length of the tubular sheath, and/or over the entire length of the introducer device.

The introducer device 3 may further comprise at least one gripping element 33 arranged at, or in the vicinity of, a non-insertable end of the introducer device. The gripping device/element 33 may e.g. be in the form of handles or flanges protruding out from the tubular sheath 31. In an embodiment, the gripping device may protrude outwardly, at least partially in a radial, lateral direction. In the illustrative embodiment of Fig. 2, two gripping devices are provided, preferably arranged separated along the circumference of the tubular sheath, and preferably at opposite sides, and protruding in opposite directions. However, only one gripping device may also be used, or more than two gripping devices. In one embodiment, the number of gripping devices/elements 33 corresponds to the number of tear lines 32 provided in the tubular sheath. In such an embodiment, the gripping devices are preferably arranged between the tear lines.

The gripping elements may be in the form of a gripping tab, a gripping ring, etc. The gripping elements may be provided with a texture, such as corrugations, to increase friction when gripped.

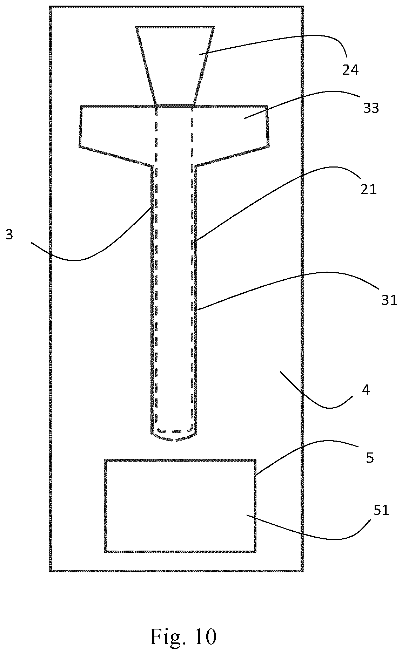

The urinary catheter assembly may further, as shown in Fig. 10, comprise a package 4 accommodating the urinary catheter 2 and the introducer device 3. The introducer device is preferably pre-arranged on the urinary catheter, thereby forming a ready-to-use kit.

The assembly may further comprise a wetting fluid 51 arranged in the package. In one embodiment, the wetting fluid is arranged in a separate compartment 5, such as a sachet, in the package 4, to be released into contact with the hydrophilic coating/surface prior to use. In another embodiment, the wetting fluid 51 is maintained in constant contact with the hydrophilic coating/surface, thereby maintaining the coating/surface in an activated, ready-to-use state.

The package may be of a flexible material, such as foil material. However, the package may also be of a more rigid material, such as rigid plastic material.

The package may, in a closed position, provide a sterile and moisture proof compartment for the introducer device and the urinary catheter.

Upon use, the catheter and introducer device are assembled together, and may e.g. be enclosed in the outer package 4, to maintain the assembly sterile. In preparation for use, the hydrophilic coating/surface of the introducer device is activated by being wetted with a wetting fluid 51, such as water or saline. The wetting fluid may be provided from an external supply, such as from a tap or the like. However, a supply of wetting fluid may also, as discussed in the foregoing, be integrated in the package. For example, a supply of wetting fluid may be arranged in a separate compartment 5 within the package, such as in a sachet, to be released into contact with the hydrophilic coating/surface immediately prior to use. In another alternative, the wetting fluid may be maintained in constant contact with the hydrophilic surface/coating during storage, thereby providing a urinary catheter assembly which is instantly ready-to-use.

When the hydrophilic coating/surface is activated, the catheter shaft is inserted into the urethra of the user together with the introducer device, while maintaining the catheter shaft in the tubular sheath of the introducer device. This stabilizes the catheter shaft during insertion, and makes it more rigid, thereby facilitating insertion and avoiding the risk of kinking and the like.

Once the catheter 2 has been inserted, the splitable tubular sheath 31 can be split and the introducer device be removed from the urethra, thereby releasing the catheter 2 and leaving it in place in the urethra.

Thus, the combination of the catheter shaft 21 and the tubular sheath 31 has increased stability and is easier to push into the urethra than what had would been the case with only the catheter shaft 21, without the introducer device 31. This also makes it possible to use a softer, more flexible catheter shaft, which makes the catheter more comfortable during use, in particular when used over an extended period of time, and which reduces the risk of pain. For example, it is hereby possible to use catheter shafts being so soft and flexible that they could not be inserted into the urethra on their own, without the assistance and stabilization from the introducer device. To this end, the catheter shaft can be made of softer materials than used conventionally, or with a thinner wall thickness. The catheter shaft can e.g. be made of silicone rubber, latex rubber, polyurethane (PUR) or thermoplastic elastomers (TPE). However, other materials are also feasible. Further, more than one material may be used, and arranged e.g. mixed to a blend or arranged in separate parts, such as in separate layers.

The introducer device, and in particular the tubular sheath, can be made of one or more plastic materials, and in particular polymer and/or thermoplastic materials, such as materials conventionally known for use as catheter shafts and the like. The tubular sheath may e.g. be formed by polyurethane, nylon, polyolefins, silicone, polyether block amide, etc. Blends of different materials may also be used, or layered structures where layers of different materials are sandwiched over each other, e.g. by co-extrusion.

The tear lines may be formed by the use of a separate material. In such embodiments, the tear line may be arranged as a string or stripe of a material which bonds poorly or loosely to a material used in the rest of the tubular sheath. For example, the tubular sheath may comprise polyphenyl ether and the stripes forming the tear lines may comprise polyurethane. However, as discussed in the foregoing, the tear lines may alternatively, or additionally, be formed mechanically, by provision of a line of decreased thickness, perforations or the like.

The introducer device, and in particular the tubular sheath, may also be made of other materials. In one embodiment, the tubular sheath may e.g. be made of a cellulose based material, such as paper. In such embodiments, the tubular sheath may be formed entirely by a cellulose based material, or alternatively, the cellulose based material may be arranged as one layer, and with a further inner and/or outer layer, e.g. made of plastic material, arranged overlying the cellulose based layer.

The urinary catheter assembly is in particular suitable for use with indwelling catheters, also referable to as retention catheters. However, it may also be used together with intermittent catheters, for short time use.

The tubular sheath is preferably made of a flexible material.

The tubular sheath may have a Shore A hardness which is greater than the Shore A hardness of the catheter shaft of the urinary catheter. In an embodiment, the tubular sheath has a Shore A hardness exceeding 65, and preferably in the range 70-90, and the catheter shaft has a Shore A hardness of 60 or lower, and preferably in the range 40-60.

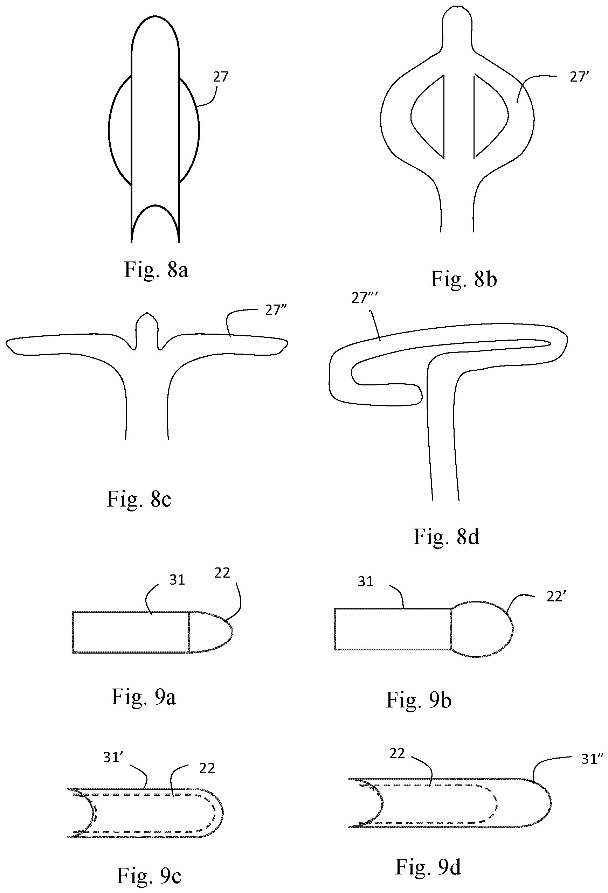

The urinary catheter 2 may further comprise an expandable retention element. The expandable retention element may take various forms, some examples of which are schematically illustrated in Figs. 8a-d. The retention element may e.g. be an inflatable element, such as an inflatable balloon 27, connected to an inflation lumen in the catheter shaft, as illustrated in Fig. 8a.

The retention element, such as in the shape of the balloon 27 in Fig. 8a, may also be made of a material which expands when not restricted, such as a foam material. In one embodiment, a balloon may be filled with such a foam material, thereby automatically inflating the balloon when a restriction around the catheter, e.g. formed by the introducer device, is released.

However, non-inflatable expandable retention elements may also be used, as e.g. schematically illustrated in Figs. 8b-d. Such non-inflatable retention elements do generally not require any inflation lumen in the catheter shaft and can consequently be used in single-lumen catheter shafts. In the embodiment of Fig. 8b, the expandable retention elements may comprise a section of the catheter shaft, close to the insertable tip, where the shaft is separated into two or more arm sections 27', such as three or four arm section, and when the arm sections when unbiased bulges outwardly, e.g. due to shape memory, and preferably in opposite directions. Alternatively, a plurality of arm sections 27" may form loose ends, each being curved outwardly from the catheter shaft, as illustrated in Fig. 8c. In another example, the entire end part 27"' of the shaft may have a curved disposition, when unbiased, such as forming a helical shape at the end of the catheter shaft, as schematically illustrated in Fig. 8d.

The introducer device is preferably arranged to maintain the expandable retention element in a biased, compressed state when accommodating the catheter shaft. Hereby, the retention element is compressed by the introducer device, for easy and simple insertion into the urethra. When the introducer device is subsequently removed, the retention element is allowed to expand, and will then serve to maintain the catheter in place.

The introducer device may protrude beyond the catheter shaft, thereby fully enclosing the insertable tip of the catheter shaft. Alternatively, the catheter shaft may protrude beyond the introducer device, thereby forming the forwardmost insertable tip of the assembly.

In one embodiment, as illustrated in Fig. 9a, the insertable end 22 of the catheter shaft extends beyond the forward end of the tubular sheath 31 of the introducer device. Hereby, the forwardmost part of the assembly is formed by the tip of the catheter shaft. The transition between the catheter shaft and the tubular sheath of the introducer device is preferably smooth and essentially flush, thereby ensuring a painless and smooth insertable part of the assembly. The length of the protruding end of the catheter shaft is preferably relatively short, thereby ensuring an easy introduction even in case no low-friction hydrophilic coating is provided on the catheter shaft.

In an alternative embodiment, to make the transition between the protruding catheter shaft and the tubular sheath of the introducer device even less noticeable for the user, the protruding end 22' of the catheter shaft may have a greater cross-sectional diameter compared to the rest of the catheter shaft, at least on a part thereof, as shown in Fig. 9b. The protruding end 22' may e.g. be formed as a ball, an oval, a tear drop, or the like. The shape may form a narrow, pointed end, gradually leading into an enlarged part, with a maximum diameter, and then continuing into a part with a somewhat smaller diameter. In such an embodiment, the tubular sheath preferably extends to a position relatively close to enlarged, maximum diameter part of the catheter shaft.

Alternatively, the introducer device may be arranged to completely enclose the insertable tip 22 of the catheter shaft. As shown in Fig. 9c, the introducer device may e.g. be arranged with a closed forward end 31' narrowly covering the insertable tip 22 of the catheter shaft. In such embodiments, the tubular sheath, with its hydrophilic coating/surface, may cover the entire insertable part of the catheter shaft, thereby facilitating insertion. In order to facilitate separation and removal of the tubular sheath, the closed forward end 31' may also be provided with tear lines, thereby facilitating severing and splitting of the tubular sheath. For example, the tear lines running along the length of the tubular sheath may extend all the way to the closed forward end 31'.

In another embodiment, as shown in Fig. 9d, the closed forward end 31" is formed by a disintegrating material. Hereby, the tip portion formed in the tubular sheath, forming the rounded forward end, may be arranged to disintegrate upon contact with water, urine or the like. For example, the forward end 31" may be arranged to start to disintegrate upon contact with water, such as when wetting the tubular sheath for activation of the hydrophilic coating/surface. The disintegration process may hereby be slow enough for the catheter to be inserted into the urethra with the tubular sheath essentially intact, and thereafter to disintegrate to facilitate removal of the tubular sheath from the catheter. Alternatively, or additionally, the forward end 31" may be arranged to disintegrate, or disintegrate more rapidly, upon contact with urine, so that the forward end 31" will disintegrate fast as soon as it reaches the bladder.

The tubular sheath 31 may further be provided with a hydrophilic coating/surface also on an inner surface. The hydrophilic coating/surface may be of the same type as the outer hydrophilic coating/surface. Such an inner coating/surface will facilitate the removal of the introducer device, since it will lower the friction between the catheter and the tubular sheath. Alternatively, another, non-hydrophilic, low friction coating/surface may be used, such as polytetrafluoroethylene (PTFE). However, alternative measures to lower the friction are also feasible, such as providing a lubricating gel or other friction reducing fluids between the introducer device and the catheter shaft.

With reference to Fig. 4, the assembly may further comprise a guide arrangement 34, such as a ring encircling the introducer device, at a position in the vicinity of the non-insertable part of the catheter. The guide arrangement 34 is preferably arranged at a non-insertable part of the assembly. The guide arrangement may e.g. be connected to the catheter, but may alternatively or additionally be connected to the tubular sheath, or other parts of the introducer device. The guide arrangement may be arranged to maintain the introducer device intact until it has passed the guide arrangement, when being pulled to be removed and split. This ensures that the splitting of the introducer device occurs after the ring, at a position outside the urethra. This is of advantage, since splitting of the introducer device may otherwise tend to occur at the urethra orifice, or to some extent inside the urethra. If this happens, it may exert stress on the urethral wall, close to the orifice, which may feel uncomfortable. However, the guiding element may also be omitted.

The tear line(s) may be weak enough to be easily splitable/severable upon pulling the gripping elements apparat by normal hand or finger force. However, to facilitate separation, and in particular for users having poor dexterity and the like, separation facilitation elements may be provided, to ensure that the separation occurs at the right place(s), and/or to lower the force required to obtain the separation.

To this end, the assembly may, as shown in Fig. 6, comprise a cutting or splitting aid 35 arranged in the vicinity of the non-insertable part of the catheter, aiding in severing the introducer device. The cutting/splitting aid 35 may e.g. be arranged connected to or integrated with the optional guide arrangement 34. The cutting/splitting aid 35 may e.g. comprise a pin, a blade or the like, penetrating into the introducer device, thereby assisting the splitting of the introducer device as it is pulled out passing the cutting aid 34.

The person skilled in the art realizes that the present invention by no means is limited to the preferred embodiments described above. On the contrary, many modifications and variations are possible within the scope of the appended claims. For example, the hydrophilic layer may be arranged as a coating, or as an integrated part of the catheter shaft. Further, introducer device may be formed of any suitable material. The urinary catheter may be a conventional urinary catheter, or be a modified catheter, having e.g. thinner catheter walls surrounding the internal lumen, be more flexible than conventional catheters, be made of a softer material than conventional catheters, etc.

Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements. Further, a single unit may perform the functions of several means recited in the claims.

## Claims

1. A urinary catheter assembly comprising a urinary catheter including a catheter shaft, and an introducer device including a splitable tubular sheath, wherein the introducer device is arranged to accommodate at least a part of the catheter shaft, and wherein at least a part of an outer surface of the tubular sheath is provided with a hydrophilic coating/surface.

2. The urinary catheter assembly of claim 1, wherein the introducer device is arranged to accommodate at least an insertable part of the catheter shaft.

3. The urinary catheter assembly of claim 1 or 2, wherein the tubular sheath has an inner diameter greater than an outer diameter of the catheter shaft, and preferably only slightly greater, and more preferably essentially corresponding to the outer diameter of the catheter shaft.

4. The urinary catheter assembly of any one of the preceding claims, wherein the introducer device comprises a first end and a second end remote from the first end, and at least one tear line extending between the first and second ends.

5. The urinary catheter assembly of claim 4, comprising at least two tear lines extending between the first and second ends, the tear lines being separated from each other in a circumferential direction.

6. The urinary catheter assembly of any one of the preceding claims, wherein the tubular sheath is made of a flexible material.

7. The urinary catheter assembly of any one of the preceding claims, wherein the introducer device further comprises at least one gripping element arranged at, or in the vicinity of, a non-insertable end of the introducer device.

8. The urinary catheter assembly of claim 7, wherein at least two gripping elements are provided, the gripping elements being separated or distributed from each other in a circumferential direction.

9. The urinary catheter assembly of claim 7 or 8, wherein the at least one gripping element is arranged to protrude outwardly, in a radial direction, from the tubular sheath.

10. The urinary catheter assembly of any one of the preceding claims, wherein the tubular sheath has a Shore A hardness which is greater than the Shore A hardness of the catheter shaft of the urinary catheter.

11. The urinary catheter assembly of claim 10, wherein the tubular sheath has a Shore A hardness exceeding 65, and preferably in the range 70-90, and the catheter shaft has a Shore A hardness of 60 or lower, and preferably in the range 40-60.

12. The urinary catheter assembly of any one of the preceding claims, wherein the urinary catheter comprises an expandable retention element, and wherein the introducer device is arranged to maintain the expandable retention element in a compressed state when accommodating the catheter shaft.

13. The urinary catheter assembly of any one of the preceding claims, wherein the catheter shaft, when accommodated in the introducer device, has an insertable tip portion protruding out from the tubular sheath of the introducer device.

14. The urinary catheter assembly of any one of the preceding claims, wherein the tubular sheath is further provided with a coating also on an inner surface, the coating preferably being a hydrophilic coating.

15. An introducer device for insertion of a urinary catheter comprising a splitable tubular sheath, wherein at least a part of an outer surface of the tubular sheath is provided with a hydrophilic coating/surface.
